# EUROPEAN PATENT APPLICATION

(11) **EP 3 058 884 A1**
(43) Date of publication of application: **24.08.2016**
(21) Application number: 14854157.6
(22) Date of filing: 06.08.2014
(51) Int. Cl.: A61B 17/221, A61B 18/14

(54) **TREATMENT TOOL MANUFACTURING METHOD AND TREATMENT TOOL**

(30) Priority: 17.10.2013 JP 2013216692
(71) Applicant: Olympus Corporation, Shibuya-ku Tokyo 151-0072 (JP)
(72) Inventor: HORII, Shingo, Hachioji-shi Tokyo 192-8507 (JP)
(74) Representative: Kuhnen & Wacker
(86) International application number: PCT/JP2014/070794
(87) International publication number: WO 2015/056480

(57) **Abstract**

In a method for manufacturing a treatment tool according to the invention, the treatment tool includes a function body, a tube portion, and an operation unit. The function body has at least one elastic wire formed of a wire material made of a super-elastic material and having a predetermined shape, and a fixing portion having an insertion hole into which the elastic wire can be inserted to house at least a proximal end portion of the elastic wire in the insertion hole so as to be fixed to the fixing portion. The tube portion is configured to house the function body. The operation unit inserts and removes the function body into and from the tube portion. The method includes an insertion step of inserting the proximal end portion of the elastic wire (6211 to 6214) into the insertion hole of the fixing portion (622), a formation step of forming, at the proximal end portion of the elastic wire (6211 to 6214), an engagement portion for engaging with the insertion hole of the fixing portion (622), and a fixing step of fixing the proximal end portion of the elastic wire (6211 to 6214) to the fixing portion (622).

## Description

### Field

The present invention relates to a method for manufacturing a treatment tool used for an endoscope, and to the treatment tool.

### Background

An endoscope system has been used in a medical field in the past to observe an organ of a subject such as a patient. As the endoscope system described above, an endoscope system has been known such as one that includes a flexible elongated insertion portion configured to be inserted into a body cavity of the subject, an image sensor provided at a distal end of the insertion portion to capture an in-vivo image, and an external device connected to the insertion portion through a cable to process the in-vivo image captured by the image sensor and display the in-vivo image on a display unit or the like. A medical doctor or the like inserts a treatment tool into the insertion portion to operate while looking at the in-vivo image captured by the image sensor, whereby a predetermined medical treatment for a subject can be performed in the inside of the subject.

Examples of the treatment tool used for the endoscope system include a wire-loop type treatment tool. The wire-loop type treatment tool has a structure in which a function body made of a wire is inserted into a tubular member in a removable manner. Specific examples of the function body include a snare whose distal end portion has a curving shape in an annular form. This wire is housed in the tubular member to reduce a diameter of a ring in size and tissue is captured to be thermally cut out by applying a high-frequency electric current to the wire.

A basket forceps is also exemplified as the function body. The basket forceps has a basket shape in which multiple wires whose central portions have curving shapes are fixed at end portions thereof while opposing wires are curved in opposite directions, and these wires are housed in the tubular member to reduce an inner space formed by the basket during the capturing. In this function body, each end portion of the plurality of wires is fixed by being brazed (for example, refer to Patent Literature 1).

Here, in recent years, in order to enhance the shape reproducibility during insertion and removal into and from the tubular member, super-elastic alloy such as nickel-titanium alloy has been employed for the wires used in the above-mentioned function bodies.

### Citation List

### Patent Literature

Patent Literature 1: JP 3629111 B2

### Summary

### Technical Problem

However, the super-elastic alloy such as nickel-titanium alloy has had a problem in that the wettability thereof makes it difficult to fix the wires through brazing or, even when the wires are fixed, heating in brazing may alter super-elastic properties such as a shape-memory property. For this reason, there is a need to fix the wires while maintaining the super-elastic properties of the wires made of super-elastic alloy.

The present invention has been made in view of the foregoing, and an object of the invention is to provide a method for manufacturing a treatment tool and to provide the treatment tool capable of fixing wires while maintaining the super-elastic properties of the wires.

### Solution to Problem

In order to solve the above described problems and achieve the object, a method for manufacturing a treatment is provided. The treatment tool includes: a function body having at least one elastic wire formed of a wire material made of a super-elastic material and having a predetermined shape, and a fixing portion having an insertion hole into which the elastic wire is insertable to house at least a proximal end portion of the elastic wire in the insertion hole so as to be fixed to the fixing portion; a tube portion configured to house the function body; and an operation unit configured to insert and remove the function body into and from the tube portion. The method according to the invention includes: an insertion step of inserting the proximal end portion of the elastic wire into the insertion hole of the fixing portion; a formation step of forming, at the proximal end portion of the elastic wire, an engagement portion for engaging with the insertion hole of the fixing portion; and a fixing step of fixing the proximal end portion of the elastic wire to the fixing portion.

In the method for manufacturing the treatment tool according to the above-described invention, the formation step includes forming the engagement portion by melting a distal end of the elastic wire using arc discharge.

In the method for manufacturing the treatment tool according to above-described invention, the formation step includes forming the engagement portion by pressing the elastic wire to change a shape of the elastic wire.

In the method for manufacturing the treatment tool according to above-described invention, the formation step includes forming the engagement portion by bending a distal end of the elastic wire.

In the method for manufacturing the treatment tool according to above-described invention, the fixing step includes sealing at least part of the insertion hole.

In the method for manufacturing the treatment tool according to above-described invention, the fixing step includes reducing a diameter of the insertion hole by applying a load thereto from outside.

In the method for manufacturing the treatment tool according to above-described invention, the fixing step includes fixing, to the fixing portion, the proximal end portion of the elastic wire, and an operation wire that is connected to the operation unit to operate a movement of the function body.

In the method for manufacturing the treatment tool according to above-described invention, the function body further includes a second fixing portion having an insertion hole into which each of a plurality of elastic wires is inserted to house other end portion of each of the plurality of elastic wires in the insertion hole so as to be fixed to the second fixing portion. The method further includes: a second insertion step of inserting the other end portion of each of the plurality of elastic wires into the insertion hole of the second fixing portion; a second formation step of forming, at the other end portion of each of the plurality of elastic wires, an engagement portion for engaging with the insertion hole of the second fixing portion; and a second fixing step of fixing the other end portion of each of the plurality of elastic wires to the second fixing portion.

In the method for manufacturing the treatment tool according to above-described invention, the second fixing step includes sealing at least part of the insertion hole.

In the method for manufacturing the treatment tool according to above-described invention, the second fixing step includes reducing a diameter of the insertion hole by applying a load thereto from outside.

In the method for manufacturing the treatment tool according to above-described invention, the insertion step further includes inserting a distal end portion of the elastic wire into the insertion hole of the fixing portion, the formation step further includes forming, at the distal end portion of the elastic wire, a second engagement portion for engaging with the insertion hole, and the fixing step includes fixing the distal end portion and the proximal end portion of the elastic wire to the fixing portion.

A treatment tool according to the invention includes: a function body having at least one elastic wire formed of a wire material made of a super-elastic material and having a predetermined shape, and a fixing portion having an insertion hole into which the plurality of elastic wires is insertable to house at least a proximal end portion of each of the elastic wires in the insertion hole so as to be fixed to the fixing portion; a tube portion configured to house the function body; and an operation unit configured to insert and remove the function body into and from the tube portion.

### Advantageous Effects of Invention

According to the present invention, an engagement portion is provided at least at one end of each of a plurality of elastic wires having super-elastic properties, and both ends of each of the plurality of elastic wires are housed in a first fixing portion or a second fixing portion so as to be fixed thereto. Therefore, it possible to fix the wires while maintaining the super-elastic properties of the wires.

### Brief Description of Drawings

FIG. 1 is a diagram illustrating a schematic configuration of an endoscope system according to a first embodiment of the invention.
FIG. 2 is a schematic view illustrating a schematic configuration of a treatment tool included in the endoscope system according to the first embodiment of the invention.
FIG. 3 is a partial cross-sectional view illustrating a configuration of main parts of the treatment tool according to the first embodiment of the invention.
FIG. 4 is a cross-sectional view illustrating the configuration of the principal portion of the treatment tool according to the first embodiment of the invention.
FIG. 5 is a cross-sectional view illustrating a cross-section of the treatment tool taken along line A-A in FIG. 4.
FIG. 6 is a cross-sectional view illustrating the configuration of the principal portion of the treatment tool according to the first embodiment of the invention.
FIG. 7 is a cross-sectional view illustrating a cross-section of the treatment tool taken along line B-B in FIG. 6.
FIG. 8 is a cross-sectional view illustrating a method for manufacturing the treatment tool according to the first embodiment of the invention.
FIG. 9 is a cross-sectional view illustrating the method for manufacturing the treatment tool according to the first embodiment of the invention.
FIG. 10 is a schematic view illustrating the method for manufacturing the treatment tool according to the first embodiment of the invention.
FIG. 11 is a cross-sectional view illustrating the method for manufacturing the treatment tool according to the first embodiment of the invention.
FIG. 12 is a cross-sectional view illustrating the method for manufacturing the treatment tool according to the first embodiment of the invention.
FIG. 13 is a cross-sectional view illustrating the method for manufacturing the treatment tool according to the first embodiment of the invention.
FIG. 14 is a diagram illustrating a configuration of main parts of a treatment tool according to a variation 1-1 of the first embodiment of the invention.
FIG. 15 is a diagram illustrating a configuration of main parts of a treatment tool according to a variation 1-2 of the first embodiment of the invention.
FIG. 16 is a cross-sectional view illustrating a configuration of main parts of a treatment tool according to a second embodiment of the invention.
FIG. 17 is a cross-sectional view illustrating a cross-section of the treatment tool taken along line C-C in FIG. 16.
FIG. 18 is a cross-sectional view illustrating a configuration of main parts of a treatment tool according to a variation 2-1 of the second embodiment of the invention.
FIG. 19 is a cross-sectional view illustrating a configuration of main parts of a treatment tool according to a third embodiment of the invention.
FIG. 20 is a diagram illustrating a configuration of main parts of a treatment tool according to a fourth embodiment of the invention.
FIG. 21 is a cross-sectional view illustrating the configuration of the principal portion of the treatment tool according to the fourth embodiment of the invention.
FIG. 22 is a cross-sectional view illustrating a method for manufacturing the treatment tool according to the fourth embodiment of the invention.
FIG. 23 is a diagram illustrating a configuration of main parts of a treatment tool according to a fifth embodiment of the invention.
FIG. 24 is a cross-sectional view illustrating the configuration of the principal portion of the treatment tool according to the fifth embodiment of the invention.
FIG. 25 is a diagram illustrating a configuration of main parts of a treatment tool according to a sixth embodiment of the invention.
FIG. 26 is a cross-sectional view illustrating the configuration of the principal portion of the treatment tool according to the sixth embodiment of the invention. Description of Embodiments

As modes for carrying out the invention (hereinafter, referred to as "embodiments"), an endoscope system for medical use that images the inside of a body cavity of a subject such as a patient to display will be described below. The embodiments are not construed to limit the invention. The same reference signs are used to designate the same elements throughout the drawings. Note that the drawings are schematic and thus a relationship between a thickness and a width of each member, ratios of the respective members, and the like are different from actual ones. In addition, portions whose dimensions or ratios are different from each other between the respective drawings are also included therein.

### (First Embodiment)

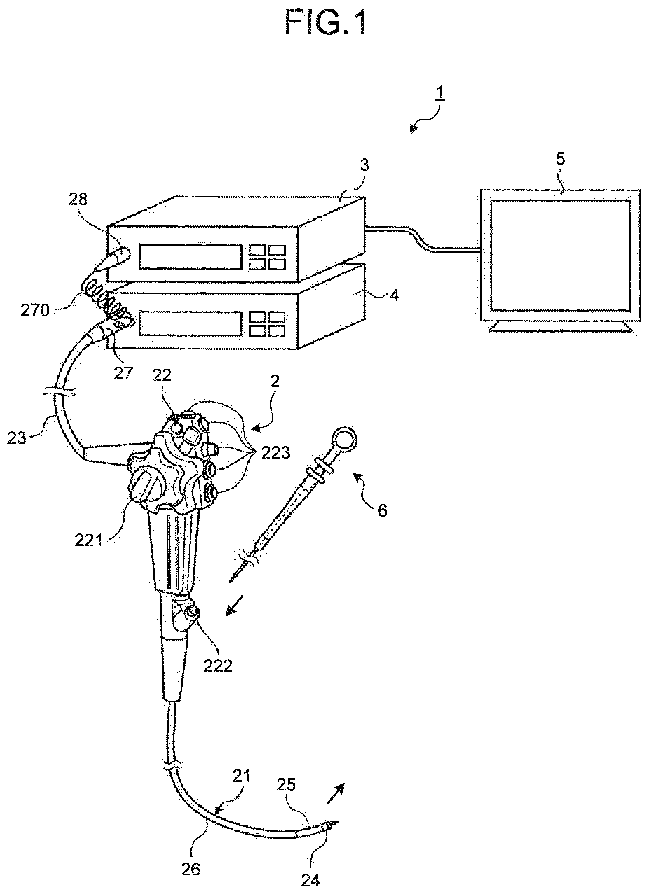

FIG. 1 is a diagram illustrating a schematic configuration of an endoscope system 1 according to a first embodiment of the invention. As illustrated in FIG. 1, the endoscope system 1 includes an endoscope 2, a control device 3, a light source device 4, a display device 5, and a treatment tool 6. The endoscope 2 captures an in-vivo image of a subject by inserting a distal end portion thereof into a body cavity of the subject. The control device 3 carries out predetermined image processing on the in-vivo image captured by the endoscope 2 and comprehensively controls overall operation of the endoscope system 1. The light source device 4 generates illumination light to be emitted from a distal end of the endoscope 2. The display device 5 displays the in-vivo image on which the control device 3 has carried out the image processing. The treatment tool 6 includes a function body such as a basket forceps or a snare at a distal end and is inserted into the endoscope 2 to be exposed from a distal end of a distal end portion 24.

The endoscope 2 includes a flexible elongated insertion portion 21, an operating unit 22 connected on a proximal end side of the insertion portion 21 to receive input of various types of operation signals, and a universal cord 23 extending from the operating unit 22 in a direction different from a direction in which the insertion portion 21 extends and incorporating various types of cables for connection to the control device 3 and the light source device 4.

The insertion portion 21 includes the distal end portion 24 having an image sensor, a bending portion 25 which is bendable and constituted by a plurality of curving pieces, and a long flexible tube portion 26 having flexibility and connected on a proximal end side of the bending portion 25.

The image sensor receives light from the outside to photoelectrically convert the light to an electric signal and carries out predetermined signal processing on the electric signal. The image sensor is realized by using, for example, a CCD image sensor or a CMOS image sensor.

A cable assembly in which a plurality of signal lines is bundled to transmit and receive the electric signals to and from the control device 3 is connected between the operating unit 22 and the distal end portion 24. The plurality of signal lines includes a signal line that transmits, to the control device 3, an image signal output by the image sensor, a signal line that transmits, to the image sensor, a control signal output by the control device 3, and the like.

The operating unit 22 includes a curving knob 221 that curves the bending portion 25 in an up-down direction and a left-right direction, a treatment tool insertion portion 222 through which the treatment tool 6 such as a biopsy forceps, a laser scalpel, or an examination probe is inserted into the body cavity, and a plurality of switches 223 serving as operation input units to which operation instruction signals for peripheral devices such as air supply means, water supply means, and gas supply means, in addition to the control device 3 and the light source device 4, are input.

The universal cord 23 incorporates at least a light guide and the cable assembly. In addition, a connector portion 27 detachable from the light source device 4, and an electrical connector portion 28 detachable from the control device 3 and electrically connected to the connector portion 27 through a coil-shaped coil cable 270, are provided at an end portion of the universal cord 23 on a side different from a side on which the operating unit 22 is connected.

Based on a picture signal output from the distal end portion 24, the control device 3 generates an in-vivo image to be displayed by the display device 5. The control device 3 carries out white balance (WB) adjustment processing, gain adjustment processing, γ correction processing, D/A conversion processing, format change processing, and the like.

The light source device 4 includes a light source, a rotary filter, a light source control unit, and the like. The light source is constituted by using a white light emitting diode (LED), a xenon lamp, or the like, and generates white light under the control of the light source control unit. The light generated by the light source is radiated from the distal end of the distal end portion 24 via the light guide.

The display device 5 has a function for receiving, from the control device 3, the in-vivo image generated by the control device 3 through an image cable to display. The display device 5 is constituted by using, for example, a liquid crystal or organic electro luminescence (EL).

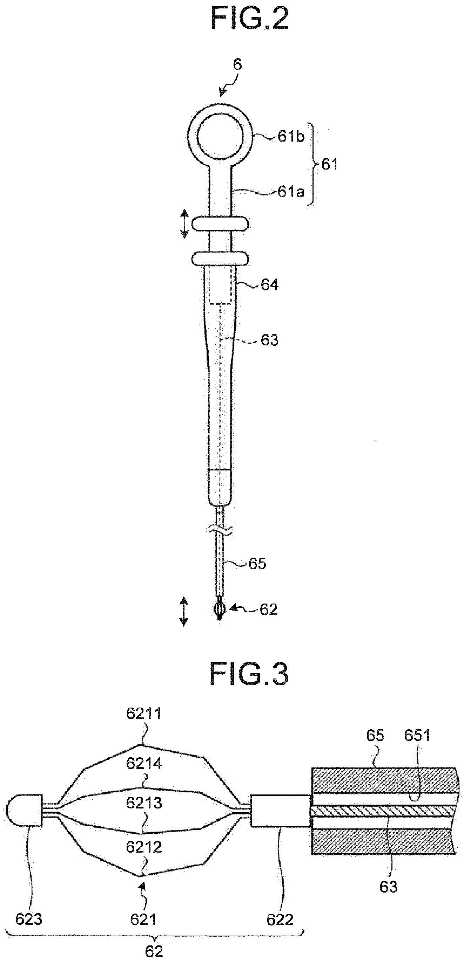

FIG. 2 is a schematic diagram illustrating a schematic configuration of the treatment tool 6 included in the endoscope system 1 according to the first embodiment. The treatment tool 6 includes a grip member 61 (operation unit), a function unit 62, an operation wire 63, a housing 64, and a flexible tube portion 65. The grip member 61 includes a substantially cylindrical base portion 61a and a grip portion 61b provided at an end portion of the base portion 61a and having an opening passing through in a direction perpendicular to a longitudinal direction of the base portion 61a. The function unit 62 includes a basket forceps as the function body. The operation wire 63 is bendable and couples the grip member 61 to the function member 62. The housing 64 has a substantially tubular shape and is configured to house the base portion 61a and the operation wire 63 such that the base portion 61a and the operation wire 63 are movable forward and backward. The flexible tube portion 65 has a tube shape and is provided at an end portion of the housing 64 on a side different from where the base portion 61a is housed. The treatment tool 6 is disposed in the endoscope 2 such that the flexible tube portion 65 is inserted through the treatment tool insertion portion 222 and a distal end of the flexible tube portion 65 can be exposed from the distal end portion 24. In the first embodiment, the operation wire 63 and the flexible tube portion 65 have flexibility in accordance with the flexibility of the insertion portion 21. The operation wire 63 and the flexible tube portion 65 may have no flexibility depending on the intended use.

The function unit 62 can be exposed to the outside from the distal end of the flexible tube portion 65 in accordance with forward and backward movement of the grip member 61 relative to the housing 64.

The operation wire 63 is formed using a wire material made of stainless steel such as SUS.

The housing 64 forms a hollow space having a stepped shape. One end of the hollow space in the housing 64 has a diameter into which the base portion 61a can be inserted and the other end thereof has a diameter into which the operation wire 63 can be inserted.

FIG. 3 is a partial cross-sectional view illustrating a configuration of main parts of the treatment tool according to the first embodiment. The function unit 62 includes a basket portion 621, a first fixing portion 622 (fixing portion), and a second fixing portion 623 (second fixing portion). The basket portion 621 forms a basket shape using a plurality of elastic wires 6211 to 6214. The first fixing portion 622 is provided on a proximal end side of the basket portion 621, collectively houses each end portion of the plurality of elastic wires 6211 to 6214 so as to be fixed, and is connected to the operation wire 63. The second fixing portion 623 is provided on a distal end side of the basket portion 621, and collectively houses each end portion of the plurality of elastic wires 6211 to 6214 so as to be fixed. The first embodiment will be described assuming that the basket shape is formed by the four elastic wires 6211 to 6214. However, the basket shape may be formed by, for example, three or five or more elastic wires.

Each of the plurality of elastic wires 6211 to 6214 is formed using a wire material made of a super-elastic material (super-elastic alloy). The super-elastic alloy is alloy that has a shape-memory property (shape-recovery property) at a temperature equal to or higher than a transformation point and has a transformation point equal to or lower than a normal temperature. For example, nickel-titanium alloy or ferro-manganese-silicon alloy can be used. The elastic wires 6211 to 6214 each have a substantially arc-shaped (C-shaped) bending shape at a central portion.

The basket portion 621 has a basket shape in which both end portions of each of the plurality of elastic wires 6211 to 6214 having the above-mentioned shape are separately fixed to the first fixing portion 622 and the second fixing portion 623 such that the C shapes of two opposing elastic wires face each other, and a plane passing through a pair of opposing elastic wires intersect a plane passing through another pair of opposing elastic wires. It is preferable that the planes passing through the elastic wires perpendicularly intersect.

The first fixing portion 622 and the second fixing portion 623 are formed using stainless steel such as SUS and fix both ends of the basket portion 621 individually such that the plurality of elastic wires 6211 to 6214 satisfies the positional relationships described above. In addition, the first fixing portion 622 has a cylindrical shape that can be inserted into a hollow portion 651 of the flexible tube portion 65. The second fixing portion 623 has a substantially cylindrical shape, where a diameter of an end portion thereof on a side different from a side on which the elastic wires 6211 to 6214 are connected (distal end portion) is made smaller toward the distal end. The hollow portion 651 has a diameter into which the first fixing portion 622 can be inserted.

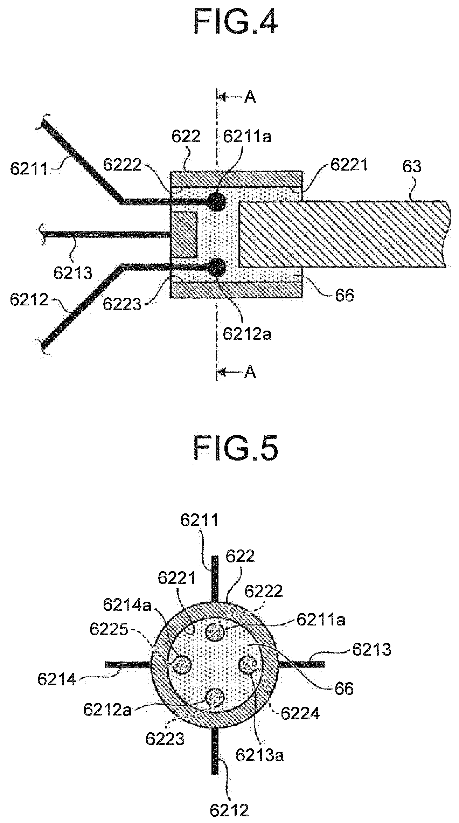

FIG. 4 is a cross-sectional view illustrating the configuration of the principal portion of the treatment tool according to the first embodiment. FIG. 5 is a cross-sectional view illustrating a cross-section of the treatment tool taken along line A-A in FIG. 4. The first fixing portion 622 includes a first hole 6221 forming a cylindrical hollow space extending from an end portion on a side on which the operation wire 63 is coupled (distal end portion) toward the other end portion, and four second holes 6222 to 6225 extending from an end portion on a side different from the distal end portion (other end portion) toward the distal end portion and communicating with the first hole 6221.

Sphere-shaped portions 6211a to 6214a serving as engagement portions, each of which has a sphere shape having a diameter equal to or larger than a diameter of the wire material of each of the elastic wires 6211 to 6214, are provided at the end portions of the elastic wires 6211 to 6214, respectively. It is further preferable that the diameter of the sphere-shaped portion be equal to or larger than, for example, a diameter of an opening of the second hole 6222.

Here, a proximal end portion of the elastic wire 6211 including the sphere-shaped portion 6211a is housed in the first hole 6221 and also inserted into the second hole 6222. The same applies to the elastic wires 6212 to 6214.

In addition, the first hole 6221 houses the end portion of the elastic wire 6211 including the sphere-shaped portion 6211a and also is filled with a sealing member 66. The second holes 6222 to 6225 are also filled with the sealing member 66. Resin such as an adhesive or solder is used as the sealing member 66. The elastic wire and the operation wire 63 are fixed in the inside of the first hole 6221 through this sealing member 66. For example, the sphere-shaped portion 6211a is fixed through the sealing member 66, whereby the elastic wire 6211 is prevented from coming off and being removed from the first hole 6221 and the second hole 6222. In addition, the elastic wires 6211 to 6214 can be prevented from moving in the inside of the first fixing portion 622.

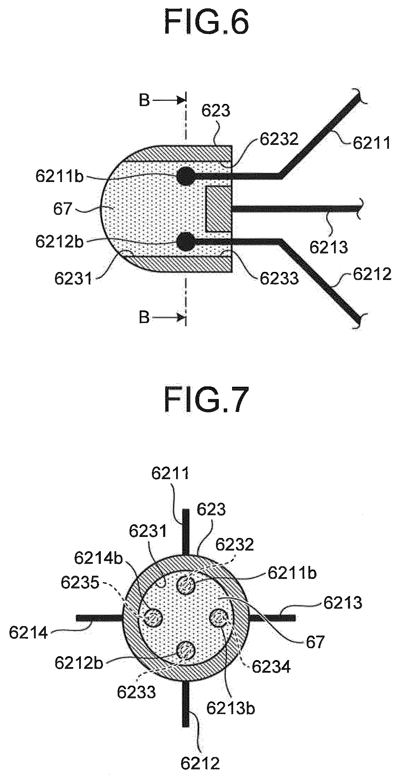

FIG. 6 is a cross-sectional view illustrating the configuration of the principal portion of the treatment tool according to the first embodiment. FIG. 7 is a cross-sectional view illustrating a cross-section of the treatment tool taken along line B-B in FIG. 6. As in the first fixing portion 622, the second fixing portion 623 also includes a first hole 6231 forming a cylindrical hollow space extending from a distal end portion toward the other end portion, and four second holes 6232 to 6235 extending from an end portion on a side different from the distal end portion (other end portion) toward the distal end portion and communicating with the first hole 6231. The first hole 6231 and the four second holes 6232 to 6235 form a through-hole (insertion hole) passing through both ends of the second fixing portion 623.

Sphere-shaped portions 6211b to 6214b (engagement portions), each of which has a sphere shape having a diameter equal to or larger than a diameter of the wire material of each of the elastic wires 6211 to 6214, are provided at the end portions of the elastic wires 6211 to 6214, respectively. It is preferable that the diameters of the sphere-shaped portions 6211b to 6214b be equal to or larger than diameters of openings of the second holes 6232 to 6235, respectively, since the sphere-shaped portions 6211b to 6214b are engaged with the second holes 6232 to 6235, respectively.

Here, the distal end portions of the elastic wires 6211 to 6214 including the sphere-shaped portions 6211b to 6214b, respectively, are housed in the first hole 6231 and also inserted into the second holes 6232 to 6235, respectively.

The first hole 6231 and the second holes 6232 to 6235 are filled with a sealing member 67. Examples of the sealing member 67 include resin such as an adhesive. Solder can be also used as long as processing can be carried out at a temperature that does not affect the shape-memory property of the elastic wire. The elastic wires 6211 to 6214 are fixed in the inside of the first hole 6231 through the sealing member 67. Each of the sphere-shaped portions 6211b to 6214b is fixed through the sealing member 67, whereby the elastic wires 6211 to 6214 are prevented from coming off and being removed from the first hole 6231 and the second holes 6232 to 6235, respectively. In addition, the elastic wires 6211 to 6214 can be prevented from moving in the inside of the second fixing portion 643.

In the treatment tool 6 having the above-mentioned configuration, in accordance with insertion and removal operation of the grip member 61 relative to the housing 64, the function unit 62 is inserted into the hollow portion 651 of the flexible tube portion 65 or removed from the hollow portion 651. When the basket portion 621 is inserted into the hollow portion 651, each of the elastic wires 6211 to 6214 is compressed in accordance with the diameter of the hollow portion 651 and made into a substantially straight line shape to be housed within the hollow portion 651. On the other hand, when the basket portion 621 is released to the outside of the hollow portion 651 through the insertion and removal operation of the grip member 61 relative to the housing 64, the elastic wires 6211 to 6214, each of which has the shape-memory property, individually form the C shapes at the central portions thereof to form the basket shape described earlier.

In the basket portion 621, the basket portion 621 first houses in-vivo tissue of the subject into the inside of the basket shape while being released to the outside of the hollow portion 651. When the grip member 61 (grip portion 61b) is moved in a direction for being removed from the housing 64, the basket portion 621 is partially housed in the hollow portion 651 to reduce an inner space formed by the basket shape, whereby the in-vivo tissue is captured and collected.

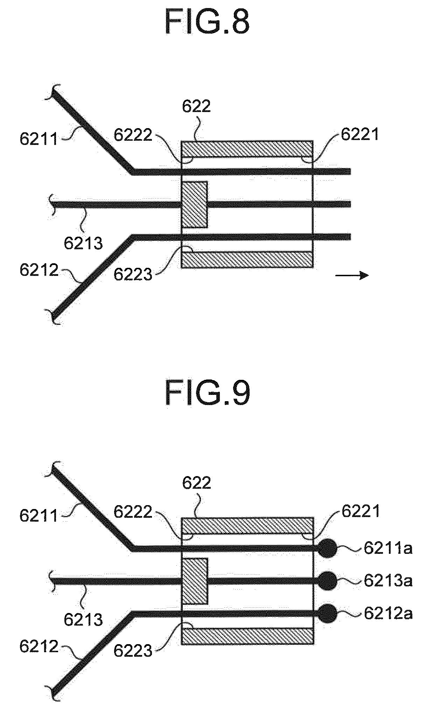

Here, a method for connecting the elastic wires 6211 to 6214 and the first fixing portion 622 will be described with reference to FIGS. 8 to 11. FIGS. 8 to 11 are diagrams illustrating a method for manufacturing the treatment tool according to the first embodiment of the invention and illustrating a method for connecting the elastic wires and the first fixing portion 622 in the function unit 62.

First, the proximal end portions of the elastic wires 6211 to 6214 are inserted into the first hole 6221 and the second holes 6222 to 6225, respectively, of the first fixing portion 622 (FIG. 8, insertion step). At this time, the proximal end portions of the elastic wires 6211 to 6214 are exposed to the outside from the first hole 6221.

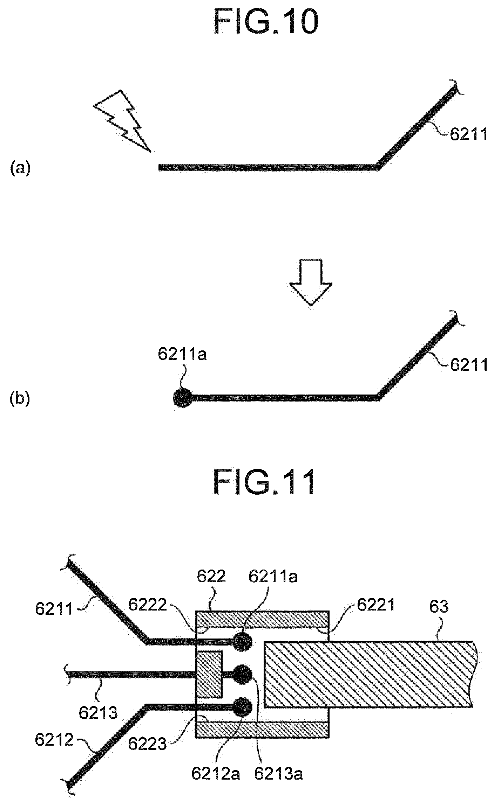

Thereafter, the sphere-shaped portions 6211a to 6214a are formed at the proximal end portions of the elastic wires 6211 to 6214, respectively (FIG. 9: formation step). For example, as illustrated in FIGS. 10(a) and 10(b), the sphere-shaped portion 6211a is formed by melting the elastic wire 6211 using arc discharge. The sphere-shaped portions 6212a to 6214a are also formed in the elastic wires 6212 to 6214, respectively, through the similar processing. In addition to the arc discharge, the elastic wire may be melted using plasma discharge. Similarly, the sphere-shaped portions 6211b to 6214b are formed at other ends of the elastic wires 6211 to 6214, respectively.

After the sphere-shaped portions 6211a to 6214a are formed in the elastic wires 6211 to 6214, respectively, the end portions of the elastic wires 6211 to 6214 are housed in the first hole 6221 (FIG. 11). As a result, the sphere-shaped portions 6211a to 6214a of the elastic wires 6211 to 6214 are disposed within the first hole 6421. Further, the operation wire 63 is partially inserted into the first hole 6221 from an opposite side of the second holes 6222 to 6225.

Thereafter, the first hole 6221 and the second holes 6222 to 6225 are filled with the sealing member 66 to be fixed (fixing step), whereby, as illustrated in FIG. 4, a structure in which the elastic wires 6211 to 6214 are fixed to the first fixing portion 622 through the sealing member 66 is obtained.

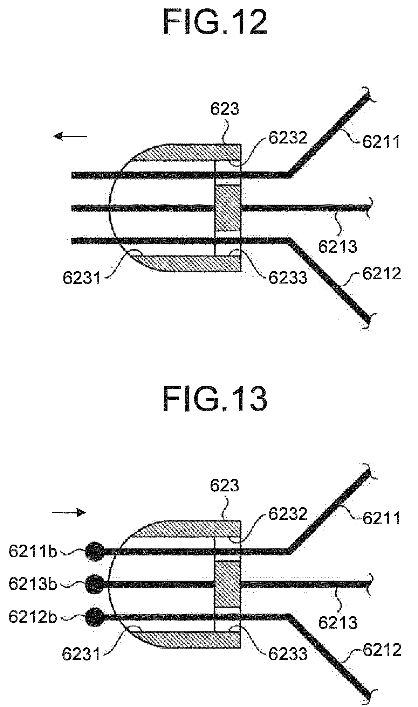

Next, a method for connecting the elastic wires 6211 to 6214 and the second fixing portion 623 will be described with reference to FIGS. 12 and 13. FIGS. 12 and 13 are diagrams illustrating the method for manufacturing the treatment tool according to the first embodiment of the invention and illustrating a method for connecting the elastic wires and the second fixing portion 623 in the function unit 62.

First, the distal end portions of the elastic wires 6211 to 6214 are inserted into the first hole 6231 and the second holes 6232 to 6235, respectively (refer to FIG. 7), of the second fixing portion 623 (FIG. 12, second insertion step). At this time, the distal end portions of the elastic wires 6211 to 6214 are exposed to the outside from the first hole 6231.

Thereafter, the sphere-shaped portions 6211b to 6214b are formed at the distal end portions of the elastic wires 6211 to 6214, respectively (FIG. 13, second formation step). The sphere-shaped portion is formed in a manner similar to the above-mentioned formation step (refer to FIGS. 10(a) and 10(b)).

After the sphere-shaped portions 6211b to 6214b are formed in the elastic wires 6211 to 6214, respectively, the end portions of the elastic wires 6211 to 6214 are housed in the first hole 6231 (FIG. 13). As a result, the sphere-shaped portions 6211b to 6214b of the elastic wires 6211 to 6214 are disposed within the first hole 6231. Thereafter, the first hole 6231 and the second holes 6232 to 6235 are filled with the sealing member 67 to be fixed (second fixing step), whereby, as illustrated in FIG. 6, a structure in which the elastic wires 6211 to 6214 are fixed to the second fixing portion 623 through the sealing member 67 is obtained.

According to the above-mentioned first embodiment, the sphere-shaped portions 6211a to 6214a (6211b to 6214b) are provided in the plurality of elastic wires 6211 to 6214, respectively, each of which has the super-elastic properties, so as to be fixed to the first fixing portion 622 (second fixing portion 623) through the sealing member 66 (67). Therefore, the wires can be fixed while the super-elastic properties of the wires are maintained.

According to the first embodiment, an adhesive (or solder) is used as the sealing members 66 and 67. Accordingly, in the process of manufacturing, the elastic wires 6211 to 6214 are prevented from losing the super-elastic properties due to heat, while the elastic wires 6211 to 6214 can be surely fixed to the first fixing portion 622 and the second fixing portion 623.

Furthermore, according to the first embodiment, the sphere-shaped portions 6211a to 6214a are provided in the plurality of elastic wires 6211 to 6214, respectively, such that the sphere-shaped portions 6211a to 6214a prevent the elastic wires from coming off from the second holes 6222 to 6225, respectively. As a result, certainty of prevention of the coming-off in the respective elastic wires 6211 to 6214 can be enhanced.

The first embodiment has been described assuming that the elastic wires 6211 to 6214 are fixed to the second fixing portion 623 using the sealing member 67. However, as long as maximum diameters of the sphere-shaped portions 6211b to 6214b are larger than opening diameters of the second holes 6232 to 6235, respectively, a configuration without the sealing member 67 can be applied. In this case, it is preferable that an opening of the first hole 6231 on a side different from a side on which the second holes are connected (outside) be sealed using a seal material or a sealing member.

The first embodiment has also been described assuming that the first hole 6221 and the second holes 6222 to 6225 are filled with the sealing member 66. However, the sealing member 66 may be provided partially in the first hole 6221 as long as the sphere-shaped portions 6211a to 6214a can be fixed (the sphere-shaped portions can be engaged with the second holes) in the first hole 6221.

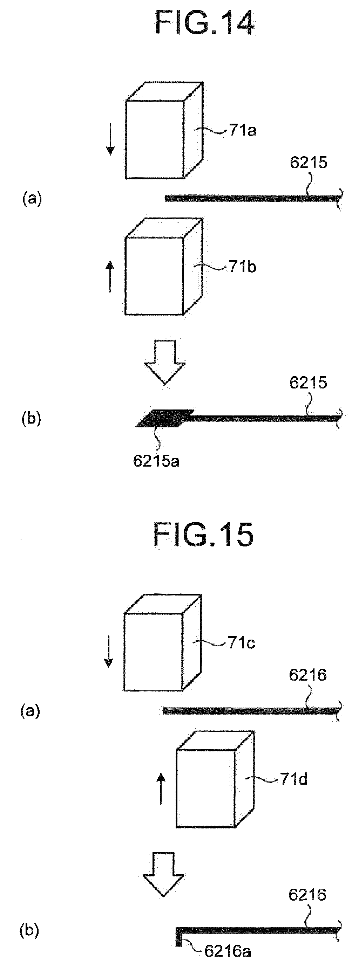

FIG. 14 is a diagram illustrating a configuration of main parts of a treatment tool according to a variation 1-1 of the first embodiment. The above-mentioned first embodiment has been described assuming that the sphere-shaped portion is formed by melting the elastic wire using the arc discharge (or plasma discharge). However, a shape of an end portion may be changed by using press jigs 71a and 71b as illustrated in FIG. 14(a) to press an elastic wire 6215. In this case, the quadrangular prism-shaped press jigs 71a and 71b are arranged such that center axes thereof in the longitudinal direction match. The end portion of the elastic wire 6215 is then placed on these center axes to be pressed.

As illustrated in FIG. 14(b), a flat plate portion 6215a (engagement portion) larger than the diameter of the wire material of the elastic wire 6215 is formed at the end portion of the elastic wire 6215, which has been pressed by the press jigs 71a and 71b. As a result, the flat plate portion 6215a has a function for preventing the coming-off from the first and second fixing portions, as in the above-mentioned sphere-shaped portion.

FIG. 15 is a diagram illustrating a configuration of main parts of a treatment tool according to a variation 1-2 of the first embodiment. The above-mentioned first embodiment has been described assuming that the sphere-shaped portion is formed by melting the elastic wire using the arc discharge (or plasma discharge). However, a shape of an end portion may be changed by using press jigs 71c and 71d as illustrated in FIG. 15(a) to press an elastic wire 6216. In this case, the quadrangular prism-shaped press jigs 71c and 71d are arranged at positions in which the press jigs 71c and 71d do not make contact with each other during vertical movement thereof by making center axes thereof in the longitudinal direction eccentric. The end portion of the elastic wire 6216 is placed between the center axes of the press jigs 71c and 71d to be pressed by the press jigs 71c and 71d.

As illustrated in FIG. 15(b), a bending portion 6216a (engagement portion) whose end portion has a shape bending substantially at right angle by being pressed by the press jigs 71c and 71d is formed at the end portion of the elastic wire 6216. As a result, the bending portion 6216a has a function for preventing the coming-off from the first and second fixing portions, as in the above-mentioned sphere-shaped portion.

### (Second Embodiment)

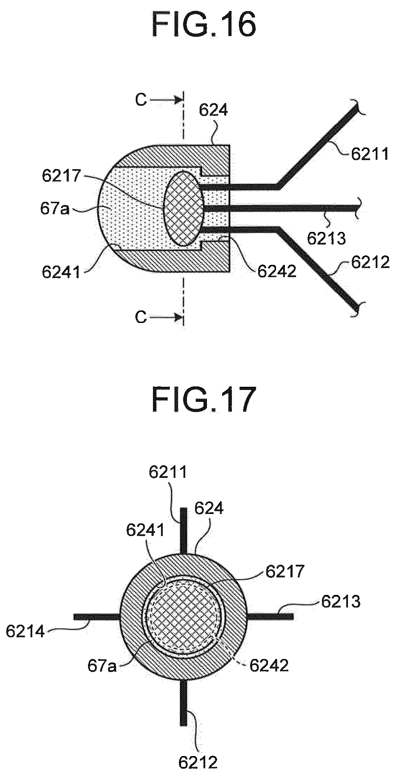

FIG. 16 is a cross-sectional view illustrating a configuration of main parts of a treatment tool according to a second embodiment of the invention. FIG. 17 is a cross-sectional view illustrating a cross-section of the treatment tool taken along line C-C in FIG. 16. Elements which are the same as those of FIG. 4 or the like are denoted by the same reference signs. The above-mentioned first embodiment has been described assuming that the sphere-shaped portion is provided in each of the plurality of elastic wires to prevent the coming-off from the second fixing portion 623. However, the second embodiment achieves an effect for preventing the coming-off from a second fixing portion by forming a bundling portion in which distal end portions of elastic wires are bundled together.

A second fixing portion 624 according to the second embodiment is formed of stainless steel such as SUS and houses a distal end portion of a basket portion 621 so as to be fixed. The second fixing portion 624 has a substantially cylindrical shape, where a diameter of an end portion thereof on a side different from a side on which the elastic wires 6211 to 6214 are connected (distal end portion) is made smaller toward the distal end.

The second fixing portion 624 includes a first hole 6241 forming a cylindrical hollow space extending from a distal end portion toward the other end portion, and a second hole 6242 extending from an end portion on a side different from the distal end portion (other end portion) toward the distal end portion, communicating with the first hole 6241, and having a diameter smaller than a diameter of an opening of the first hole 6241. A center axis of the opening of the first hole 6241 matches a center axis of an opening of the second hole 6242, and a through-hole (insertion hole) whose cross-section has a stepped shape is formed.

At the distal end portion of the plurality of elastic wires 6211 to 6214, a bundling portion 6217 (engagement portion) in which the respective elastic wires 6211 to 6214 are bundled together is provided. Here, a maximum diameter of the bundling portion 6217 is larger than the diameter of the opening of the second hole 6242. As a result, the bundling portion 6217 has a function for preventing the second fixing portion 624 from coming off the second hole 6242, as in the above-mentioned sphere-shaped portion.

Here, the elastic wires 6211 to 6214, which has been subjected to processing similar to the above-mentioned method (FIGS. 12 and 13) to form the bundling portion 6217, are then fixed to the second fixing portion 624. At this time, the first hole 6241 and the second hole 6242 are filled with a sealing member 67a. Resin such as an adhesive or solder is used as the sealing member 67a. As a result, the elastic wires 6211 to 6214 can be prevented from moving in the inside of the second fixing portion 624.

According to the above-mentioned second embodiment, the bundling portion 6217 is provided in the plurality of elastic wires 6211 to 6214 having the super-elastic properties so as to be fixed to the second fixing portion 624 through the sealing member 67a. Therefore, the wires can be fixed while the super-elastic properties of the wires are maintained.

Additionally, according to the second embodiment, the bundling portion 6217 in which the plurality of elastic wires 6211 to 6214 is bundled together is provided, whereby processing steps for the elastic wires 6211 to 6214 can be reduced compared to the above-mentioned first embodiment.

The second embodiment has been described assuming that the elastic wires 6211 to 6214 are fixed to the second fixing portion 624 using the sealing member 67a. However, a configuration without the sealing member 67a can be applied. In this case, it is preferable that the opening of the first hole 6241 on a side different from a side on which the second hole 6242 is connected (outside) be sealed using a seal material or a sealing member.

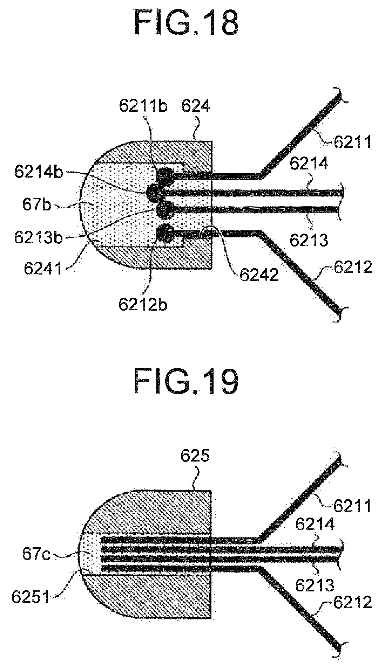

FIG. 18 is a cross-sectional view illustrating a configuration of main parts of a treatment tool according to a variation 2-1 of the second embodiment. The above-mentioned elastic wires 6211 to 6214 having the sphere-shaped portions 6211b to 6214b, respectively, according to the first embodiment may be connected to the second fixing portion 624 described above.

In this case, when the sphere-shaped portions 6211b to 6214b are closely provided on the same plane and, when, for example, relative to one plane, the sphere-shaped portions 6211b to 6214b are arranged such that respective centers thereof are placed on this plane and circles including cross-sections of the sphere-shaped portions 6211b to 6214b are minimized in area on this plane, the sphere-shaped portions 6211b to 6214b are formed so as to be larger in area than the opening of the second hole 6242.

The respective sphere-shaped portions 6211b to 6214b are fixed through a sealing member 67b. In a case where, as illustrated in FIG. 18, the sphere-shaped portions 6211b to 6214b are not placed on the same plane, it is preferable that positions where the sphere-shaped portions are formed be adjusted at the distal ends of the elastic wires.

The above-mentioned first and second embodiments have been described assuming that the elastic wires 6211 to 6214 are fixed to the first and second fixing portions using the sealing members 66, 67, 67a, and 67b. However, instead of the sealing member, swaging in which a diameter of a through-hole is reduced by being applied with a load from the outside may be employed to pinch and fix elastic wires.

### (Third Embodiment)

FIG. 19 is a cross-sectional view illustrating a configuration of main parts of a treatment tool according to a third embodiment of the invention. Elements which are the same as those of FIG. 3 or the like are denoted by the same reference signs. The above-mentioned first embodiment has been described assuming that the sphere-shaped portions 6211b to 6214b are provided in the elastic wires 6211 to 6214, respectively, to prevent the coming-off from the second fixing portion 623. However, the third embodiment achieves an effect for preventing a plurality of elastic wires from coming off a second fixing portion through solder or an adhesive.

A second fixing portion 625 according to the third embodiment is formed of stainless steel such as SUS and houses one end of a basket portion 621 so as to be fixed. The second fixing portion 625 has a substantially cylindrical shape, where a diameter of an end portion thereof on a side different from a side on which the elastic wires 6211 to 6214 are connected (distal end portion) is made smaller toward the distal end. In addition, a hole portion 6251 (insertion hole) forming a cylindrical hollow space extending from the distal end portion toward the other end portion is formed in the second fixing portion 625.

Here, the elastic wires 6211 to 6214 are housed in the hole portion 6251 and then fixed to the second fixing portion 625. At this time, the hole portion 6251 is filled with a sealing member 67c. Resin such as an adhesive or solder is used as the sealing member 67c. As a result, the elastic wires 6211 to 6214 can be prevented from moving in the inside of the second fixing portion 625 or coming off the second fixing portion 625.

According to the above-mentioned third embodiment, one end of each of the plurality of elastic wires 6211 to 6214 having the super-elastic properties is housed in the second fixing portion 625 such that the elastic wires are fixed to the second fixing portion 625 by being filled with the sealing member 67c. Therefore, the wires can be fixed while the super-elastic properties of the wires are maintained.

### (Fourth Embodiment)

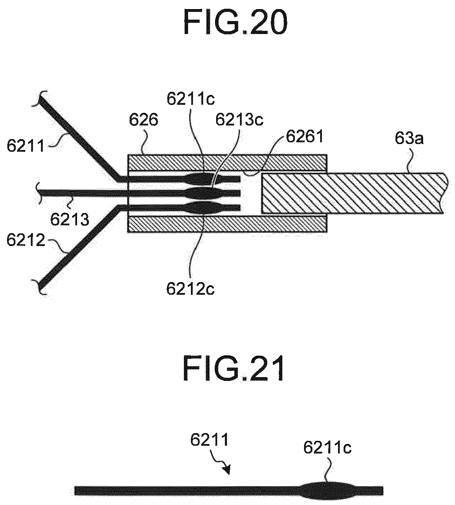

FIGS. 20 and 21 are diagrams illustrating a configuration of main parts of a treatment tool according to a fourth embodiment of the invention. Elements which are the same as those of FIG. 3 or the like are denoted by the same reference signs. The above-mentioned first embodiment has been described assuming that the sphere-shaped portion 6211a or the like is provided in each of the elastic wires 6211 to 6214 to prevent the coming-off from the first fixing portion 622. However, the fourth embodiment achieves an effect for preventing the coming-off by forming an engagement portion at each proximal end portion of a plurality of elastic wires to fix by swaging a first fixing portion.

A substantially cylindrical first fixing portion 626 according to the fourth embodiment is formed of stainless steel such as SUS and houses the other end of a basket portion 621 and an operation wire 63a so as to be fixed. In addition, a hole portion 6261 (insertion hole) forming a cylindrical hollow space extending from a distal end portion toward the other end portion is formed in the first fixing portion 626.

A flat plate-shaped flat plate portion 6211c formed by changing a shape thereof through pressure so as to have a maximum diameter equal to or larger than the diameter of the wire material of the elastic wire 6211 is provided at an end portion (other end) of the elastic wire 6211 (refer to FIG. 21). Likewise, the flat plate portions are also formed in the elastic wires 6212 to 6214 (FIG. 20 illustrates flat plate portions 6212c and 6213c formed in the elastic wires 6212 and 6213, respectively).

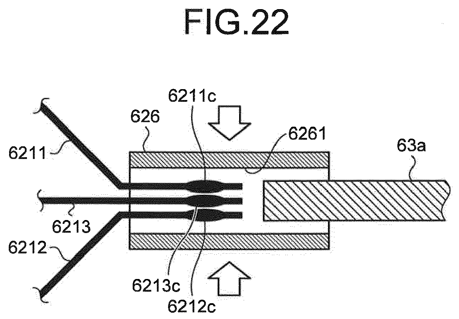

FIG. 22 is a cross-sectional view illustrating a method for manufacturing the treatment tool according to the fourth embodiment. For example, the elastic wires 6211 to 6214 are housed in the hole portion 6261 of the first fixing portion 626 and then fixed by swaging the first fixing portion 626. Specifically, a load is applied to the first fixing portion 626 from a direction substantially perpendicular to a center axis of the cylindrical shape (center axis of the hole portion 6261) to reduce a diameter of an outer circumference thereof in size. In accordance with this, a diameter of the hole portion 6261 is also reduced in size. When the diameter of the hole portion 6261 is reduced in size, the flat plate portions (6211c to 6213c) of the elastic wires 6211 to 6214 and an end portion of the operation wire 63a are squeezed by the hole portion 6261. As a result, the elastic wires 6211 to 6214 can be prevented from moving in the inside of the first fixing portion 626 or coming off the first fixing portion 626.

Here, it is preferable that a diameter of the operation wire 63a be equal to or larger than a diameter of an outer circumference of a pile obtained by collecting all of the flat plate portions of the elastic wires 6211 to 6214 together.

According to the above-mentioned fourth embodiment, the other end of each of the plurality of elastic wires 6211 to 6214 having the super-elastic properties is housed in the first fixing portion 626, and then the first fixing portion 626 is swaged so that the other end of each of the elastic wires 6211 to 6214 is fixed. Therefore, the wires can be fixed while the super-elastic properties of the wires are maintained.

In the above-mentioned first to fourth embodiments, a mode for fixing both of the ends of the elastic wires 6211 to 6214 is not limited to a combination according to each of the first to fourth embodiments. The treatment tool can be configured such that both of the ends are fixed by combining the respective fixing modes according to the first to fourth embodiments independently. In addition, the respective fixing modes for the first and second fixing portions described above can be selected as necessary for the first and second fixing portions. For example, the fixing mode for the first fixing portion can be replaced with the fixing mode for the second fixing portion. The respective fixing modes can be applied to any configuration as long as the engagement portions are formed at least at one ends of the elastic wires 6211 to 6214 and both ends of the elastic wires 6211 to 6214 are fixed by the first and second fixing portions.

### (Fifth Embodiment)

FIGS. 23 and 24 are diagrams illustrating a configuration of main parts of a treatment tool according to a fifth embodiment of the invention. Elements which are the same as those of FIG. 3 or the like are denoted by the same reference signs. The above-mentioned first embodiment has been described assuming that the function unit 62 includes the basket forceps as the function body. However, in the fifth embodiment, a function unit includes a snare as the function body.

The function unit 62a according to the fifth embodiment includes an annular-shaped portion 627 and a fixing portion 622a. The annular-shaped portion 627 has a substantially annular shape and makes both end portions of a single elastic wire 6271 come close to each other such that a central portion of the elastic wire 6271 has a predetermined radius of curvature. The fixing portion 622a collectively houses both of the end portions of the elastic wire 6271 so as to be fixed and is connected to an operation wire 63.

The elastic wire 6271 is formed using a wire material made of a super-elastic material (super-elastic alloy). The super-elastic alloy is alloy that has a shape-memory property (shape-recovery property) at a temperature equal to or higher than a transformation point and has a transformation point equal to or lower than a normal temperature. For example, nickel-titanium alloy or ferro-manganese-silicon alloy can be used.

The fixing portion 622a is formed using stainless steel such as SUS and fixes both ends (a distal end portion and a proximal end portion) of the annular-shaped portion 627 thereto. The fixing portion 622a also has a cylindrical shape that can be inserted into a hollow portion 651 of a flexible tube portion 65.

The fixing portion 622a includes a first hole 6221 forming a cylindrical hollow space extending from an end portion on a side on which the operation wire 63 is coupled (distal end portion) toward the other end portion, and two second holes 6226 and 6227 communicating with the first hole 6221 and each forming an opening at an end portion on a side different from the distal end portion (proximal end portion).

At both of the end portions of the elastic wire 6271 (the proximal end portion and the distal end portion), sphere-shaped portions 6271a and 6271b (engagement portions), each of which has a sphere shape having a diameter equal to or larger than a diameter of the wire material, are provided. It is further preferable that the diameter of the sphere-shaped portion be equal to or larger than, for example, a diameter of the opening of each of the second holes 6226 and 6227.

Here, the proximal end portion of the elastic wire 6271 including the sphere-shaped portion 6271a is housed in the first hole 6221 and also inserted into the second hole 6226. Meanwhile, the distal end portion of the elastic wire 6271 including the sphere-shaped portion 6271b is housed in the first hole 6221 and also inserted into the second hole 6227.

Both of the end portions of the elastic wire 6271 (sphere-shaped portions 6271a and 6271b) housed in the first hole 6221 are fixed to the fixing portion 622a through a sealing member 66a. A conductive adhesive such as solder is used as the sealing member 66a. For example, the sphere-shaped portion 6271a is fixed through the sealing member 66a, whereby the elastic wire 6271 is prevented from coming off and being removed from the first hole 6221 and the second hole 6226. In addition, the elastic wire 6271 can be prevented from moving in the inside of the fixing portion 622a.

In accordance with insertion and removal operation of a grip member 61 relative to a housing 64, the function unit 62a is inserted into the hollow portion 651 of the flexible tube portion 65 or removed from the hollow portion 651. When the annular-shaped portion 627 is inserted into the hollow portion 651, the elastic wire 6271 is compressed in accordance with the diameter of the hollow portion 651 and made into a substantially straight line shape to be housed within the hollow portion 651. On the other hand, when the annular-shaped portion 627 is released to the outside of the hollow portion 651 through the insertion and removal operation of the grip member 61 relative to the housing 64, a shape of the elastic wire 6271 having the shape-memory property is changed so as to have a predetermined radius of curvature and the shape is recovered to the annular shape described earlier.

The annular-shaped portion 627 first houses in-vivo tissue of the subject into the inside of the annular shape while being released to the outside of the hollow portion 651. When the grip member 61 is moved in a direction for being removed from the housing 64, the annular-shaped portion 627 is partially housed in the hollow portion 651 to reduce an inner space formed by the annular-shaped portion 627, whereby the in-vivo tissue is captured. At this time, a high-frequency electric current is applied to the elastic wire 6271 through the operation wire 63 and a cable (not illustrated) and then heat is generated through the electrification. The captured in-vivo tissue can be cut out using the heat.

In the treatment tool (function unit 62a) according to the fifth embodiment, the proximal end portion of the elastic wire 6271 is inserted into the second hole 6226 in the fixing portion 622a while the distal end portion thereof is inserted into the second hole 6227. Thereafter, the sphere-shaped portions 6271a and 6271b are each formed therein and then the elastic wire 6271 is fixed through the sealing member 66a.

According to the above-mentioned fifth embodiment, the sphere-shaped portions 6271a and 6271b are provided at both of the end portions of the elastic wire 6271 having the super-elastic properties so as to be fixed to the fixing portion 622a through the sealing member 66a. Therefore, both of the end portions of the wire can be fixed while the super-elastic properties of the wire are maintained.

### (Sixth Embodiment)

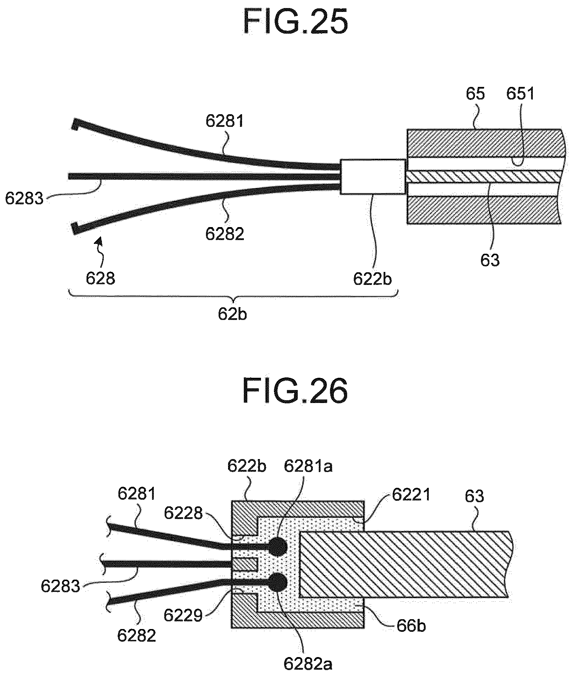

FIGS. 25 and 26 are diagrams illustrating a configuration of main parts of a treatment tool according to a sixth embodiment of the invention. Elements which are the same as those of FIG. 3 or the like are denoted by the same reference signs. The above-mentioned first embodiment has been described assuming that the function unit 62 includes the basket forceps as the function body. However, in the sixth embodiment, a function unit includes a tripod grasping forceps as the function body.

The function unit 62b according to the sixth embodiment includes a tripod portion 628 and a fixing portion 622b. The tripod portion 628 is formed by three elastic wires 6281 to 6283 having predetermined shapes to have a shape in which a space surrounded by the elastic wires 6281 to 6283 is radially expanded from a proximal end portion side toward a distal end portion side. The fixing portion 622b collectively houses the proximal end portions of the elastic wires 6281 to 6283 of the tripod portion 628 so as to be fixed and is connected to an operation wire 63.

The elastic wires 6281 to 6283 are formed using a wire material made of a super-elastic material (super-elastic alloy). The super-elastic alloy is alloy that has a shape-memory property (shape-recovery property) at a temperature equal to or higher than a transformation point and has a transformation point equal to or lower than a normal temperature. For example, nickel-titanium alloy or ferro-manganese-silicon alloy can be used.

The fixing portion 622b is formed using stainless steel such as SUS and fixes the proximal end portion of the tripod portion 628 thereto. The fixing portion 622b also has a cylindrical shape that can be inserted into a hollow portion 651 of a flexible tube portion 65.

The fixing portion 622b includes a first hole 6221 forming a cylindrical hollow space extending from an end portion on a side on which the operation wire 63 is coupled (distal end portion) toward the other end portion, and three second holes (FIG. 26 only illustrates the second holes 6228 and 6229) communicating with the first hole 6221 and forming an opening at an end portion on a side different from the distal end portion (proximal end portion).

At the proximal end portions of the elastic wires 6281 to 6283, sphere-shaped portions (Only the sphere-shaped portions 6281a and 6282a are illustrated in FIG. 26) serving as engagement portions, each of which has a sphere shape having a diameter equal to or larger than a diameter of the wire material, are provided. It is further preferable that the diameter of the sphere-shaped portion be equal to or larger than, for example, a diameter of the opening of the second hole.

Here, the proximal end portions of the elastic wires 6281 to 6283 including the sphere-shaped portions are housed in the first hole 6221 and also inserted into the second holes individually.

The proximal end portions of the elastic wires 6281 to 6283 housed in the first hole 6221 are fixed to the fixing portion 622b through a sealing member 66b. Resin such as an adhesive or solder is used as the sealing member 66b. For example, the sphere-shaped portion 6281a is fixed through the sealing member 66b, whereby the elastic wire 6281 is prevented from coming off and being removed from the first hole 6221 and the second hole 6228. In addition, the elastic wire 6281 can be prevented from moving in the inside of the fixing portion 622b.

In accordance with insertion and removal operation of a grip member 61 relative to a housing 64, the function unit 62b is inserted into the hollow portion 651 of the flexible tube portion 65 or removed from the hollow portion 651. When the tripod portion 628 is inserted into the hollow portion 651, the elastic wires 6281 to 6283 are compressed in accordance with the diameter of the hollow portion 651 and made into a substantially straight line shape to be housed within the hollow portion 651. On the other hand, when the tripod portion 628 is released to the outside of the hollow portion 651 through the insertion and removal operation of the grip member 61 relative to the housing 64, shapes of the respective elastic wires 6281 to 6283 having the shape-memory property are changed so as to have the predetermined shapes.

The tripod portion 628 first encloses in-vivo tissue of the subject on the distal end portion side of the elastic wires 6281 to 6283 while being released to the outside of the hollow portion 651. When the grip member 61 is moved in a direction for being removed from the housing 64, the tripod portion 628 is partially housed in the hollow portion 651 to reduce the space surrounded by the elastic wires 6281 to 6283, whereby the in-vivo tissue is captured and collected.

According to the above-mentioned sixth embodiment, the sphere-shaped portions are provided at the proximal end portions of the respective elastic wires 6281 to 6283 having the super-elastic properties so as to be fixed to the fixing portion 622b through the sealing member 66b. Therefore, the wires can be fixed while the super-elastic properties of the wires are maintained.

The above-mentioned first to sixth embodiments are merely examples for carrying out the invention and are not construed to limit the invention. For example, the invention can be applied to any treatment tool formed using a wire material made of a super-elastic material and used by fixing at least a proximal end of an elastic wire having a predetermined shape. Additionally, the various inventions can be made by combining multiple elements disclosed in the respective embodiments or variations as necessary. It is clear from the description above that the invention can be variously modified depending on specifications or the like, and furthermore, other different embodiments can be made within the scope of the invention.

### Industrial Applicability

As described above, the method for manufacturing the treatment tool as well as the treatment tool according to the invention are useful for fixing the wires while maintaining the super-elastic properties of the wires. Reference Signs List

1 ENDOSCOPE SYSTEM
2 ENDOSCOPE
3 CONTROL DEVICE
4 LIGHT SOURCE DEVICE
5 DISPLAY DEVICE
6 TREATMENT TOOL
21 INSERTION PORTION
22 OPERATING UNIT
23 UNIVERSAL CORD
24 DISTAL END PORTION
25 BENDING PORTION
26, 65 FLEXIBLE TUBE PORTION
27 CONNECTOR PORTION
28 ELECTRICAL CONNECTOR PORTION
61 GRIP MEMBER
61a BASE PORTION
61b GRIP PORTION
62, 62a, 62b FUNCTION MEMBER
63, 63a OPERATION WIRE
64 HOUSING
66, 66a, 66b, 67, 67a, 67b, 67c SEALING MEMBER
71a, 71b, 71c, 71d PRESS JIG
221 CURVING KNOB
222 TREATMENT TOOL INSERTION PORTION
223 SWITCH
621 BASKET PORTION
622, 626 FIRST FIXING PORTION
622a, 622b FIXING PORTION
623, 624, 625 SECOND FIXING PORTION
627 ANNULAR-SHAPED PORTION
628 TRIPOD PORTION
651 HOLLOW PORTION
6211 to 6214, 6215, 6216, 6271, 6281 to 6283 ELASTIC WIRE
6211a to 6214a, 6211b to 6214b, 6271a, 6271b, 6281a, 6282a SPHERE-SHAPED PORTION
6215a, 6211c, 6212c, 6213c FLAT PLATE PORTION
6216a BENDING PORTION
6217 BUNDLING PORTION
6221, 6231, 6241 FIRST HOLE
6222 to 6229, 6232 to 6235, 6242 SECOND HOLE
6251, 6261 HOLE PORTION

## Claims

1. A method for manufacturing a treatment tool, the treatment tool comprising: a function body having at least one elastic wire formed of a wire material made of a super-elastic material and having a predetermined shape, and a fixing portion having an insertion hole into which the elastic wire is insertable to house at least a proximal end portion of the elastic wire in the insertion hole so as to be fixed to the fixing portion; a tube portion configured to house the function body; and an operation unit configured to insert and remove the function body into and from the tube portion,
the method comprising:
an insertion step of inserting the proximal end portion of the elastic wire into the insertion hole of the fixing portion;
a formation step of forming, at the proximal end portion of the elastic wire, an engagement portion for engaging with the insertion hole of the fixing portion; and
a fixing step of fixing the proximal end portion of the elastic wire to the fixing portion.

2. The method for manufacturing the treatment tool according to claim 1, wherein
the formation step includes forming the engagement portion by melting a distal end of the elastic wire using arc discharge.

3. The method for manufacturing the treatment tool according to claim 1, wherein
the formation step includes forming the engagement portion by pressing the elastic wire to change a shape of the elastic wire.

4. The method for manufacturing the treatment tool according to claim 1, wherein
the formation step includes forming the engagement portion by bending a distal end of the elastic wire.

5. The method for manufacturing the treatment tool according to any one of claims 1 to 4, wherein
the fixing step includes sealing at least part of the insertion hole.

6. The method for manufacturing the treatment tool according to any one of claims 1 to 4, wherein
the fixing step includes reducing a diameter of the insertion hole by applying a load thereto from outside.

7. The method for manufacturing the treatment tool according to any one of claims 1 to 6, wherein
the fixing step includes fixing, to the fixing portion, the proximal end portion of the elastic wire, and an operation wire that is connected to the operation unit to operate a movement of the function body.

8. The method for manufacturing the treatment tool according to any one of claims 1 to 7, wherein
the function body further comprises a second fixing portion having an insertion hole into which each of a plurality of elastic wires is inserted to house other end portion of each of the plurality of elastic wires in the insertion hole so as to be fixed to the second fixing portion,
the method further comprising:
a second insertion step of inserting the other end portion of each of the plurality of elastic wires into the insertion hole of the second fixing portion;
a second formation step of forming, at the other end portion of each of the plurality of elastic wires, an engagement portion for engaging with the insertion hole of the second fixing portion; and
a second fixing step of fixing the other end portion of each of the plurality of elastic wires to the second fixing portion.

9. The method for manufacturing the treatment tool according to claim 8, wherein
the second fixing step includes sealing at least part of the insertion hole.

10. The method for manufacturing the treatment tool according to claim 8, wherein
the second fixing step includes reducing a diameter of the insertion hole by applying a load thereto from outside.

11. The method for manufacturing the treatment tool according to any one of claims 1 to 7, wherein
the insertion step further includes inserting a distal end portion of the elastic wire into the insertion hole of the fixing portion,
the formation step further includes forming, at the distal end portion of the elastic wire, a second engagement portion for engaging with the insertion hole, and
the fixing step includes fixing the distal end portion and the proximal end portion of the elastic wire to the fixing portion.

12. A treatment tool comprising:
a function body having at least one elastic wire formed of a wire material made of a super-elastic material and having a predetermined shape, and a fixing portion having an insertion hole into which the plurality of elastic wires is insertable to house at least a proximal end portion of each of the elastic wires in the insertion hole so as to be fixed to the fixing portion;
a tube portion configured to house the function body; and
an operation unit configured to insert and remove the function body into and from the tube portion.
